(19) Europäisches Patentamt
European Patent Office
Office européen des brevets

(11) Publication number: **0 518 263 A1**

# (12) EUROPEAN PATENT APPLICATION

(21) Application number: **92109699.6**

(22) Date of filing: **09.06.92**

(51) Int. Cl.5: **A61K 31/52**, A61K 9/50, A61K 9/54, A61K 9/52

(30) Priority: **10.06.91 US 712479**

(43) Date of publication of application: **16.12.92 Bulletin 92/51**

(84) Designated Contracting States: **AT BE CH DE DK ES FR GB GR IT LI LU NL PT SE**

(71) Applicant: **HOECHST-ROUSSEL PHARMACEUTICALS INCORPORATED**
**P.O. Box 2500, Route 202-206**
**Somerville, NJ 08876-1258(US)**

(72) Inventor: **Urban, Joseph**
**5 James Drive**
**Richboro, Pennsylvania 18954(US)**
Inventor: **Henderson, Norman**
**6 Patriot Road**
**Gladstone, NJ 07934(US)**
Inventor: **Wood, Alan**
**9 Norwich Place**
**Somerset, NJ 08873(US)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

## (54) Micropellets and a process for their manufacture.

(57) Micropellets of a medicament, binder, and coating characterized by exhibiting a controlled release of medicament determined by the number of discrete layers of coating and the size of the pellets in a pharmaceutical dosage from and a process for the preparation of the micropellets is disclosed.

EP 0 518 263 A1

The present invention relates to micropellets. More particularly, the present invention relates to micropellets comprising a medicament, a binder, and a discrete number of layers of a coating exhibiting a controlled release of the medicament determined by the number of discrete layers of coating in a pharmaceutical dosage form and a process for the preparation thereof.

Several pharmaceutical preparations in the form of beadlets containing a medicament surrounded by a protective coating are known in the art. For example, J. P. Aterno, et al., U.S. Patent 2,811,483 granted October 29, 1957 describes beadlets of a vitamin and/or mineral-coated seed encased in a sealing material, which are readily administered as such or as sprinkles on food products, C. H. Hsiao, U.S. Patent 4,587,118 granted May 6, 1986 discloses micropellets of theophylline formed by inner seeds coated with theophylline and polyvinylpyrrolidone that are protected by a coat of ethyl cellulose and hydroxypropyl cellulose to provide a sustained release formulation, and W. Fulberth and E. Neitzer. U.S. 3,835,221 granted September 10, 1974 discusses a delayed action dosage form consisting of sugar globules or nonpareils coated with a medicament and overlaid with a release delaying coating of polyvinyl acetate and ethyl cellulose having a continuous rate of release of the active ingredient.

H. Lowey, U.S. Patent 2,853,420 granted September 23, 1958 describes a granulated carrier coated with successive layers of a medicament containing a cellulose compound to provide a bead having predetermined action delaying or action sustaining effect.

It has now been found that sequential coating of a granulation of a medicament, preferably a water soluble medicament, and a binder with a release retarding coating such as a cellulose derivative affords a micropellet having controlled release characteristics determined by the number of discrete layers of coating. The micropellets can be formulated in pharmaceutical unit dosage forms in containers such as capsules blisters, or packetts for sprinkling on foods thereby providing a means of administration of a unit dosage of a medicament to patients, who generally find it very difficult to swallow tablets, capsules, and even liquids. It has also been found that these characteristics may be enhanced and determined by screening the granulation, collecting the defined granules, coating the screened granules, and performing the screening and collecting procedure between successive coatings.

The micropellets of the present invention comprise a particulate core of medicament and a binder, the resulting granule being encased in a release retarding coating. Among medicaments suitable as particulate cores of micropellets, there may be mentioned sedatives such as, e.g., flurazepam, antiulcer compuonds such as, e.g., ranitidine, antiinflammatories such as, e.g., naproxen, antiinfectives such as, e.g., doxycycline, central nervous system stimulants such as, e.g., amphetamine, antihistamines such as, e.g.. diphenhydramine, antiarrhythmics such as, e.g., nifedipine, antihypertensives such as, e.g., ramipril, antiepileptics such as, e.g., valproic acid, skeletomuscular relaxants such as, e.g., cyclobenzaprine, antidiabetics such as, e.g., tolbutamide, diuretics such as, e.g., furosemide, bronchodilators such as, e.g., salbutanol, expectorants such as, e.g., albuterol, antitussives such as, e.g., dextromethorphan, decongestants such as, e.g, pseudoephedrine, and vasodilators such as xanthines of the formula

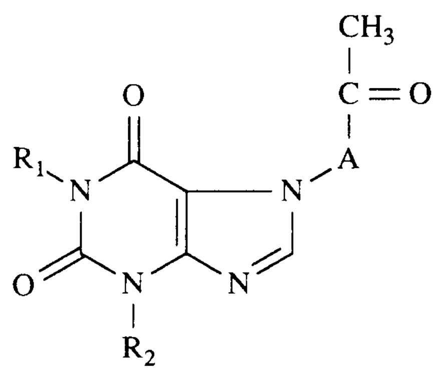

in which $R_1$ and $R_2$ are the same or different and are independently selected from the group consisting of straight-chain or branched-chain alkyl radicals with 2 to 6 carbon atoms, cyclohexyl, straight chain or branched chain alkoxyalkyl, and hydroxyalkyl radicals; and A is a hydrocarbon radical with up to 4 carbon atoms which can be substituted by a methyl group, and

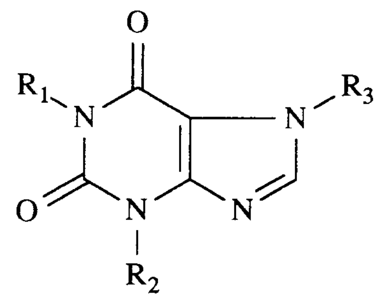

wherein at least one of $R_1$ and $R_3$ is either (a) a branched hydroxyalkyl group of the formula

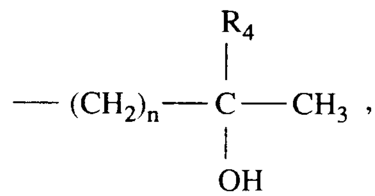

with a tertiary alcohol function, in which $R_4$ stands for an alkyl group with 1 to 3 carbon atoms and n stands for a whole number from 2 to 5, the other $R_1$ or $R_3$ group that may optionally be present stands for a hydrogen atom or an aliphatic hydrocarbon group $R_5$ with up to 6 carbon atoms, whose carbon chain may be interrupted by up to 2 oxygen atoms or may be substituted with a hydroxy or oxo group, or (b) at least one of $R_1$ or $R_3$ is an oxoalklyl group of the formula

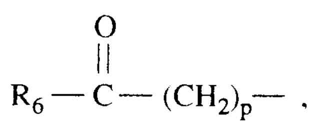

wherein $R_6$ is $C_1$-$C_6$ alkyl, and p is 2, 3 or 4; the other $R_1$ or $R_3$ group being defined as above and $R_2$ represents an alkyl group with 1 to 4 carbon atoms.

Preferred are vasodilating xanthines of the formula

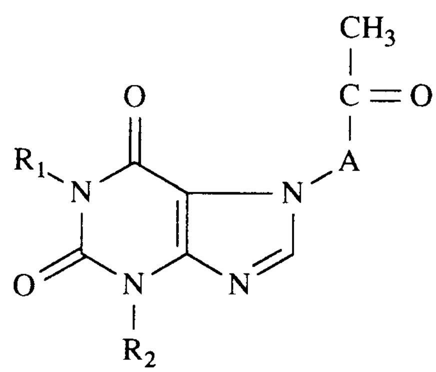

wherein $R_1$, $R_2$, and A are as above. Particularly preferred is pentoxifylline, i.e., a xanthine of the formula wherein $R_1$ is $CH_3CO(CH_2)_4$ and $R_2$ and $R_3$ are methyl.

Water soluble medicaments are preferred in the practice of the present invention. Water insoluble medicaments may be utilized in the form of their water soluble derivatives such as salts of basic and acidic medicaments, i.e., for example, hydrohalides (hydrobromides, hydrochlorides, etc.) and alkali metal salts (sodium, potassium, salts, etc.) of, respectively, amino or carboxylic acid bearing medicaments.

Suitable binders include polymers such as starches, for example, pregelatinized starch, celluloses, such as, for example, ethyl cellulose and cellulose acetate, and oligosaccharides, such as, for example, sucrose, and polyvinylpyrrolidone. Polyvinylpyrrolidone is the preferred binder. Polyvinylpyrrolidone having a molecular weight within the range of about 28,000 to 90,000 is most preferred, whereas polyvinylpyrrolidone having a weight of about 90,000 is particularly preferred.

Suitable coatings comprise polymers, i.e. synthetic polymers, such as alkyl celluloses, for example, methyl cellulose, ethyl cellulose, and the like Ethyl cellulose is preferred.

Optimally, in addition to the binder and coating, a primary plasticizer, a secondary plasticizer, a coating processing aid and a solvent may be incorporated in the casing of the micropellet. Preferred primary and secondary plasticizers are dibutyl sebacate and oleic acid, respectively, coating processing aid is fumed silica, and the solvent is aqueous ammonia, which together with ethyl cellulose is available from Colorcon, a Division of Berwind Pharmaceutical Services, Inc. of West Point, Pennsylvania under the name Surelease as a plasticized dispersion of ethyl cellulose in water. While a micropellet comprising a binder and a coating, in particular ethyl cellulose, provides a useful micropellet, it is desirable to incorporate the plasticizers, coating, processing aids, and a solvent; it is most desirable to incorporate the ingredients of the commercially available Surelease.

To provide a formulation of micropellets in a container for use as sprinkles, to be added to a food substance, the size of the pellet should fall within the range of about 0.42 to about 3.36 millimeters, preferably about 0.42 to about 1.19 millimeters. Micropellets falling within these ranges are easily seen on food, readily swallowed, and conveniently contained in a container such as a capsule, blister, or packett, easily opened by such patients.

The size of the ultimate micropellet is defined by conventional screening techniques. Thus, for example, to obtain a micropellet having a size between 0.42 and 1.19 millimeters, for example 0.59 millimeters, one mechanically shakes the sample through a series of successively smaller sieves, starting with a 1.19 millimeter sieve and finally using a 0.42 millimeter sieve. The desired ultimate micropellet size may also be obtained by successive screening between coatings using appropriate sieves. As hereinafter discussed, the rate of release of the medicament from the micropellet depends not only on the number of successively applied layers of a coating to a granule, but also upon the size of the coated pellet. By proper adjustment of the pellet size and the number of coating layers, control of the rate of release of the medicament is achieved.

The amount of medicament in each micropellet is determined by the required dose and the number of pellets per container. Typically the weight percent of medicament in each pellet to obtain the desired dose in a conveniently sized container is from about 52% to about 92% of the weight of the pellet, preferably about 72%.

The weight percent of binder to micropellet is not narrowly critical; it is desired, however, for pellet stability to employ about 1% to about 6% of binder for each pellet. A weight percent of about 3% of binder is preferred to obtain the desired stability.

While the rate of release of medicament from a micropellet is determined by the number of layers of coating (and the size of the pellet), controlled release of medicament to achieve desired blood levels of medicament is obtained by employing a total of about 7% to about 46% of coating to weight of micropellet.

The micropellets of the present invention are formulated by processes comprising operations conventional in the pharmaceutical arts. In a typical process, a solution of a polymer is sprayed onto a medicament and the granulation is screened, dried and coated, the drying and coating steps being repeated at least one additional time or as many times as may be necessary to obtain the desired release rate. Alternatively, the oversized screened granulation may be milled, screened, and coated and dried as described above.

An advantageous process for the preparation of a micropellet according to the invention consists in granulating a mixture of a medicament and a binder, drying, screening and coating the granulation and repeating the steps of drying, screening and coating and optionally milling the granulation.

Another process for the preparation of a micropellet comprises the steps of:

(a) granulating a mixture of a medicament and a polymer;
(b) drying the granulation of step (a);
(c) screening the dried granulation of step (b);
(d) collecting the screened granulation of step (c);
(e) coating the collected granulation of step (d);
(f) drying the coated granulation of step (e);
(g) coating the coated granulation of step (f);
(h) optionally, repeating steps (f) and (g).

Another process for the preparation of a micropellet comprises the steps of:

(a) granulating a mixture of a medicament and a polymer;
(b) drying the granulation of step (a);
(c) screening the dried granulation of step (b);

(d) collecting the screened granulation of step (c);
(e) milling the collected granulation of step (d);
(f) collecting the milled granulation of step (e);
(g) coating the milled granulation of step (f);
(h) drying the coated granulation of step (g);
(i) recoating the coated granulation of step (h);
(j) optionally, repeating steps (h) and (i).

Another process for the preparation of a micropellet comprises the steps of:
(a) granulating a mixture of a medicament and a polymer;
(b) drying the granulation of step (a);
(c) milling the dried granulation of step (b);
(d) separating the milled granulation of step (c);
(e) coating the separated granulation of step (d);
(f) drying the coated granulation of step (e);
(g) recoating the separated granulation of step (f);
(h) optionally, repeating the steps (f) and (g).

Another process for the preparation of a micropellet comprises the steps of:
(a) granulating a mixture of a medicament and a polymer;
(b) drying the granulation of step (a);
(c) screening the dried granulation of step (b);
(d) collecting the screened granulation of step (c);
(e) coating the separated granulation of step (d);
(f) drying the coated granulation of step (e);
(g) recoating the coated granulation of step (f);
(h) optionally, repeating steps (f) and (g).

Generally a polymer, for example, polyvinylpyrrolidone, preferably a polyvinylpyrrolidone having a molecular of about 28,000 to about 90,000, most preferably a molecular weight of about 90,000, is mixed with a solvent, preferably water, most preferably purified water, in a suitable vessel and stirred until dissolution or dispersion is complete. The solution of polymer is sprayed onto a medicament, preferably a xanthine of the formulas

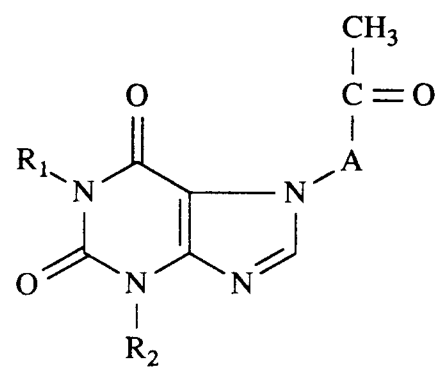

wherein $R_1$, $R_2$ and A are as above, most preferably pentoxifylline, i.e., a compound in which $R_1$ and $R_2$ are the same or different and are independently selected from the group consisting of straight-chain or branched-chain alkyl radicals with 2 to 6 carbon atoms, cyclohexyl, straight or branched chain alkoxyalkyl, and hydroxyalkyl radicals; and A is a hydrocarbon radical with up to 4 carbon atoms which can be substituted by a methyl group,

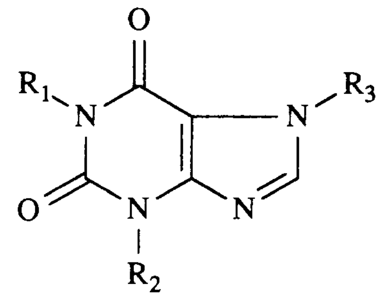

wherein at least one of $R_1$ and $R_3$ is either (a) a branched hydroxyalkyl group of the formula

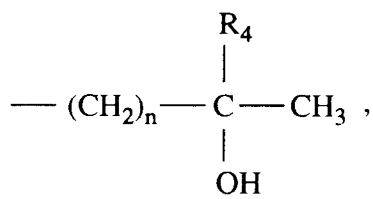

with a tertiary alcohol function, in which $R_4$ stands for an alkyl group with 1 to 3 carbon atoms and n stands for a whole number from 2 to 5, the other $R_1$ or $R_3$ group that may optionally be present stands for a hydrogen atom or an aliphatic hydrocarbon group $R_5$ with up to 6 carbon atoms, whose carbon chain may be interrupted by up to 2 oxygen atoms or may be substituted with a hydroxy or oxo group, or (b) at least one of $R_1$ or $R_3$ is an oxoalklyl group of the formula

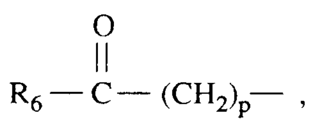

wherein $R_6$ is $C_1$-$C_6$ alkyl, and p is 2, 3 or 4; the other $R_1$ or $R_3$ group being defined as above and $R_2$ represents an alkyl group with 1 to 4 carbon atoms, and

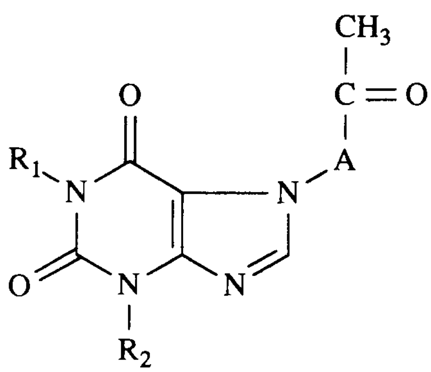

wherein $R_1$, $R_2$, and A are as above, most preferably pentoxifylline, i.e., a compound wherein $R_1$ is $CH_3CO$-$(CH_2)_4$ and $R_2$ and $R_3$ are methyl suspended by air at an incoming temperature of from about 50°C to about 90°C, preferably at about 70° to 80°C in a fluid bed granulator with an input air velocity of about 60% to about 70%, preferably an air velocity of about 60 to about 65% and an atomization air pressure of about 3.0 to about 6.0 bars, an air pressure of about 4 to about 5 bars being preferred. The granulation is most preferably performed at an incoming air temperature of from about 75°C, an air velocity of about 62%, and atomization air pressure of about 4.5 bars, a solution spray rate of about 2 to about 3 liters/minute and a granulation time of about 50 to about 60 minutes, with drying, to provide a dried granulation. The dried granulation is screened by shaking through a series of successively smaller sieves and selecting the

portions having a micromesh size compatible with the number of sequential layers of coating to afford the desired release rate. Granules having a size of about 0.42 millimeters to about 3.36 millimeters being preferred and those having a size of about 0.59 millimeters to about 2.0 millimeters being most preferred.

The coating is applied to the dried, screened granulation by spraying a solution of a release retardant, preferably a cellulose such as ethyl cellulose, in a solvent such as water, preferably purified water, onto the dried, screened granules in a fluid bed granulator under conditions similar to those employed in the initial granulation step. Preferred conditions for the coating procedure are (a) incoming air temperature of about 50° to about 80° C, preferably an air temperature of about 60° to about 70° C, (b) air velocity of about 50 to about 75%, preferably about 60 to about 65%, (c) atomization of about 3 to about 6 bars, preferable about 4 to about 5 bars, (d) solution feed rate of about 1 to about 4 liters/minute, preferably a feed rate of about 3 liters/minute, and (e) a granulation time of about 40 to about 70 minutes, preferably a time of about 50 to about 60 minutes, followed by drying. The dried, coated micropellets are recoated and dried by repeating this procedure, at least one additional time, or until the targeted release rate is determined.

The alternative milling step in the formulation of the micropellets of the present invention involves screening the dried granules of medicament and polymer as hereinbefore described and grinding and cutting the oversized granules until they pass through a sieve of the desired size and using separated granules in the coating step.

The number of layers of a coating is from about 2 to about 25.

While the micropellets prepared as described hereinbefore are usable as sprinkles on food for the administration of a medicament, an additional coating of a cellulose, for example, hydroxypropylmethyl cellulose in a solvent consisting of polyethylene glycol, benzyl alcohol and purified water may be employed.

A plurality of micropellets comprising a medicament, a binder, and a discrete number of layers of a coating in an easily openable container provide a controlled, determinable dosage unit for administration of a medicament. To administer the dosage unit of the medicament, the container is opened and the micropellets are sprinkled onto a food substance in solid or liquid form. To distinguish the micropellets from the food substance, the micropellets should be sufficiently large so that they are discernible. The pellets should also be sufficiently small so that they are swallowed without difficulty. The size of the micropellets, to satisfy these criteria, is preferably from about 0.42 to about 3.36 millimeters, most preferably 0.59 to 2.0 millimeters.

The amount of medicament necessary to provide a dose suitable for the intended uses of the pellets of the present invention depends upon the number of micropellets contained in each container. While the number of micropellets per container is not narrowly critical, about 2,300 micropellets per capsule is optimal. At the optimum number of micropellets, each pellet contains about 0,43 % of the desired dose.

DETERMINATION OF DISSOLUTION RATES

Dissolution: The test is performed on 6 sample packetts using the following conditions:
Apparatus: USP Apparatus 2 (paddles)
Conditions:

| | |
|---|---|
| Dissolution Medium: | distilled water, deaerated with helium |
| Medium Temperature: | 37 ± 0.5° C |
| Medium Volume: | 900 mL |
| Paddle Speed: | 100 rpm |
| Sampling intervals: | granules: 1, 4, 8, and 12 hours |

Standard Preparation: The standard and check standard are prepared in duplicate. Pentoxifylline RS (about 75 mg) is weighed accurately into a 100-mL volumetric flask and dissolved in and diluted to volume with water. The aqueous solution (4.0 mL) is pipetted into a 200-mL volumetric flask and diluted to volume with water. The concentration of pentoxifylline is approximately 0.015 mg/mL.

Sample Preparation: One packett is emptied into each of six dissolution vessels (i.e. one packett/dissolution vessel) as quickly as possible. A spatula or brush is used to transfer any beads which may stick to the packett.

Procedure:

White granules: Aliquots (10.0 mL) are removed from the dissolution medium at the times indicated and

are filtered or centrifuged. The clear solution (5.0 mL) is pipetted into a 100-mL volumetric flask and diluted to volume with water.

Alternatively, the SMI-automated dilutor is used and the clear solution (500 µl) is diluted with 9,500 µl of water.

Orange granules: Aliquots (10.0 mL) are removed from the dissolution medium at the times indicated and filtered or centrifuged. The clear solution (8.0 mL) is pipetted into a 500-mL volumetric flask and diluted to volume with water (the sample solution).

Alternatively, the SMI-automated dilutor is used and the solution is diluted twice:

i. The clear solution (500 µL) is diluted with 9,500 µL of water; or

ii. This solution (3,333 µL) is diluted with 6,667 µL of water (the sample solution).

Measurements:

Using a suitable spectrophotometer the absorbances of the sample and standard solutions are measured at 274 nm in 1-cm cells using water as a blank.

The % Standard Check is calculated by the following formula:

$$\frac{Astd \times Wchstd \times 100}{Achstd \times Wstd} = \%\ Standard\ Check$$

where Astd and Achstd equals the absorbances of the standard and check standards, respectively. Wstd and Wchstd equals the weights of the standard and check standard, respectively.

The % Check Standard equals 100.0 ± 2.0, or a third standard is similarly prepared and analyzed to determine which of the previously prepared standards was acceptable.

Calculation:

a. Computer Calculation: The Apple IIE personal computer with in-house dissolution program (W.P. Rogers version 84.2) is used.

b. Manual Calculation:

Granules for the one hour samples:

$$T_1 = \frac{(Aspl/Astd) \times Cstd \times D \times V \times 100}{L}$$

where Aspl and Astd equals the absorbances of the sample and standard, respectively; Cstd is the concentration of the standard; D is a dilution factor; V is volume of dissolution medium; and L is label claim.

The micropellets for the dissolution determination are prepared as described below:

PREPARATIONS OF MICROPELLETS FOR DISSOLUTION RATE DETERMINATIONS

A suspension of polyvinylpyrrolidone and purified water (about 2.5 to 4.0% polyvinylpyrrolidone solids) is sprayed onto pentoxifylline in an air suspension coator-drier. The granulation is dried and screened. The particles having a size of 0.42 to 1.19 millimeters are collected. Those larger than 1.19 millimeters are milled (0.8 mm) and screened, again collecting the 0.42 to 1.19 millimeter particles. These particles are coated with Surelease (a mixture containing ethyl cellulose, 15% solids in purified water) and allowed to dry to ambient temperature. The dried particles are recoated with Surelease and dried. The dissolution (%) determined at 1 hour and at 4 hours for the first and second coatings as a function of the amount of Surelease (% solids) is shown in Table 1.

TABLE 1

| Coating | Surelease (% solids) | Dissolution (%) | |
|---|---|---|---|
| | | 1 Hr. | 4 Hr. |
| first | 15<br>22.5 | 28.9<br>33.2 | 61.4<br>75.2 |
| first<br>second<br>second | 25<br>17.5 (total)<br>27.5 (total) | 26.0<br>24.2<br>25.7 | 66.7<br>52.0<br>64.6 |

Dissolution (%) of medicament pentoxifylline was also determined at several intervals (hours) applying Surelease for a total of 27.5% solids in five sequences of 5%, 10%, 15%, 20%, and 27.5% and two sequences of 22.5% and 27.5%, respectively. The dissolution (%) at several intervals is shown in Table 2.

TABLE 2

| Dissolution (%) (Five Coatings) | Time (Hr.) | Dissolution (%) (Two Coatings) |
|---|---|---|
| 5.0 | 1 | 25.7 |
| 9.9 | 2 | 42.8 |
| 18.9 | 4 | 64.6 |
| 36.9 | 8 | 86.0 |
| 47.7 | 12 | 94.0 |
| 62.7 | 16 | 97.3 |
| 73.4 | 24 | 100.5 |

The results presented in Tables 1 and 2 establish the nexus between the amount of coating, as solids, and the number of discrete coatings that are used to coat the granule. Thus, when granules are coated with 15% and 2.5% solids in two discrete steps to give a granule coated with 17.5% Surelease solids and with 22.5% and 5% solids to give granules coated with 27.5% solids, the dissolutions at 1 hour of 24.2% and 25.7% are the same, within experimental error. Furthermore, application of a 2.5% Surelease solid to a granule having an original coating of 15% causes approximately the same decrease in dissolution 5% and 9%, and 7% and 10% at 1 hour and 4 hours, respectively, as the application of 5.0% Surelease solids to a granule originally coated with 22.5% Surelease solids.

In addition, the results presented in Table 2 show that the dissolution of medicament is independent of the total amount of coating, being a function, instead, of the number of discrete layers of applied coating. Thus, the dissolution of medicament is markedly different at all intervals for a total amount of solid when the coating is applied in five layers or in two layers, the dissolution being considerably slower when multiple layers are applied.

EXAMPLE

Preparation of Pentoxifylline Micropellets

Polyvinylpyrrolidine (Povidone, USP) (6.18 kg) and purified water (82.1 kg) are mixed for 60 min at 25°C. Pentoxifylline (200 kg) is charged in a fluid bed granulator and suspended with incoming air at 70°C, air velocity at 60-65%, atomization at 4 bars, and exhaust air at 30.0-31.5°C. The polyvinylpyrrolidine solution is sprayed into the fluid bed granulator at incoming air temperature of 70°C, air velocity of 65%, atomization at 4 bars, exhaust air at 41°C, solution feed rate at 1.3 liters/min, granulating time of 60 minutes and cycle 60 sec (spray) and 15 sec (shake).

The granulated material is collected and screened through a Sweco, Model LS-18533 sifter. The 0.42 to 1.19 millimeter cut is collected and the oversized granules milled and screened in a Glatt Quick Sieve , the 0.42 to 1.19 millimeter cut being collected to give 125.1 kg of granules, having 51.4 kg fines.

The 0.42 to 1.19 millimeter granules are loaded into a fluid bed granulator and sprayed with a uniform suspension of 25% Surelease (183 kg) and purified water (USP) (120 kg) mixed for a minimum of 15

minutes at room temperature and incoming air temperature of 70 ° C, air velocity in of 68%, atomization air of 4 bars, exhaust air temperature of 40 ° C, solution feed rate of 1.3 liters/minute, and total granulating time of 8 hours to give 154.18 kg of coated micropellets in five stops of 100 liters.

A solution of hydroxypropylmethyl cellulose (Type E-5) (3.727 kg), polyethylene glycol (6000-8000) NF (0.207 kg), benzyl alcohol NF (0.466 kg) and purified water USP (35.587 kg) is sprayed onto 150.18 kg of the coated granules in a fluid bed granulator at an incoming air temperature of 70 ° C, air velocity in of 65%, atomization air of 4 bars, exhaust air temperature of 40 ° C, solution feed rate of 1.3 liters/minutes, to provide 154.413 kg of coated, sealed micropellets.

## Claims

1. A micropellet comprising a medicament, a binder, and a discrete number of layers of a coating.

2. A micropellet according to claim 1, wherein the medicament is a water-soluble medicament.

3. A micropellet according to claims 1 or 2, wherein the water-soluble medicament is a compound of the formulas

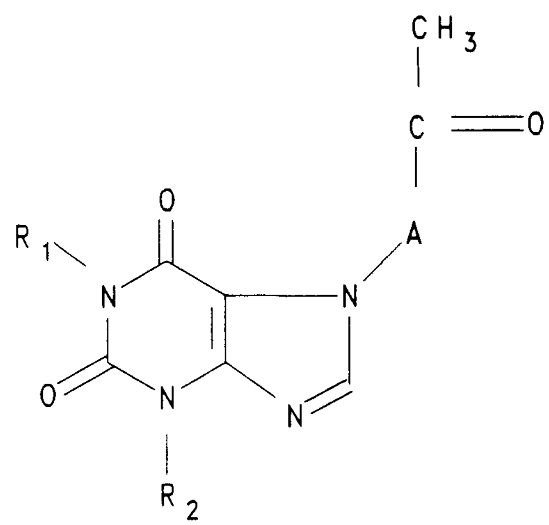

in which $R_1$ and $R_2$ are the same or different and are independently selected from the group consisting of straight-chain or branched-chain alkyl radicals with 2 to 6 carbon atoms, cyclohexyl, straight-chain or branched-chain alkoxyalkyl, and hydroxylalkyl radicals, and A is a hydrocarbon radical with up to 4 carbon atoms which can be substituted by a methyl group, and

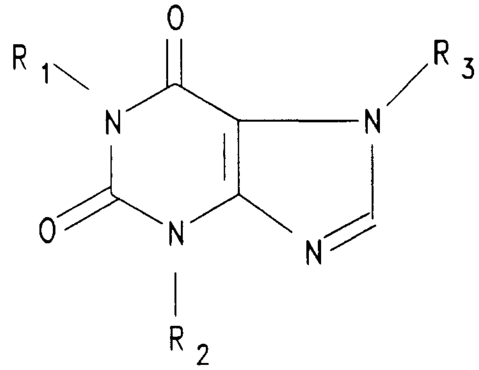

wherein at least one of $R_1$ and $R_3$ is either (a) a branched hydroxyalkyl group of the formula

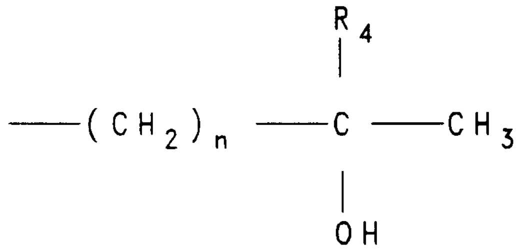

with a tertiary alcohol function, in which $R_4$ stands for an alkyl group with 1 to 3 carbon atoms and n stands for a whole number from 2 to 5,
the other $R_1$ or $R_3$ group that may optionally be present stands for a hydrogen atom or an aliphatic hydrocarbon group $R_5$ with up to 6 carbon atoms, whose carbon chain may be interrupted by up to 2 oxygen atoms or may be substituted with a hydroxy or oxo group, or
(b) at least one of $R_1$ or $R_3$ is an oxoalkyl group of the formula

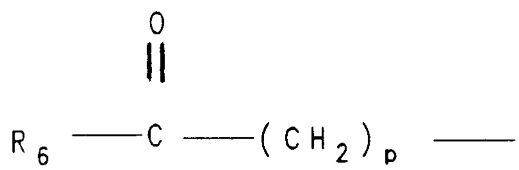

wherein $R_6$ is $C_1$-$C_6$ alkyl, and p is 2, 3 or 4;
the other $R_1$ or $R_3$ being defined as above and $R_2$ represents an alkyl group with 1 to 4 carbon atoms.

**4.** The micropellet according to claim 3, wherein the compound is pentoxifylline.

**5.** A micropellet according to one or more of claims 1 to 4, wherein the binder is a polymer selected from the group consisting of polyvinylpyrrolidone, a starch, a cellulose, and an oligosaccharide.

**6.** A micropellet according to one or more of claims 1 to 5, wherein the number of discrete layers of a coating is from about 2 to about 25.

**7.** A micropellet according to one or more of claims 1 to 6, having a pellet size of about 0.42 millimeters to about 3.36 millimeters.

**8.** A micropellet according to one or more of claims 1 to 7, wherein the weight of medicament is from about 52 % to about 92 % of the weight of the micropellet.

**9.** A controlled, determinable release dosage formulation comprising an openable container containing a plurality of micropellets according to one or more of claims 1 to 8, the micropellets in the capsule constituting a dosage unit.

**10.** A process for the preparation of a micropellet according to one or more of claims 1 to 9, which comprises the steps of granulating a mixture of a medicament and a binder, screening and coating the granulation and repeating the steps of drying, screening and coating and optionally milling the granulation.

**Claims for the following Contracting States: ES, GR**

**1.** A process for the preparation of a micropellet comprising a medicament, a binder, and a discrete number of layers of a coating which comprises the steps of granulating a mixture of a medicament and a binder, drying, screening and coating the granulation and repeating the steps of drying, screening and coating and optionally milling the granulation.

2. A process according to claim 1, wherein the medicament is a water-soluble medicament.

3. A process according to claims 1 or 2, wherein the water-soluble medicament is a compound of the formulas

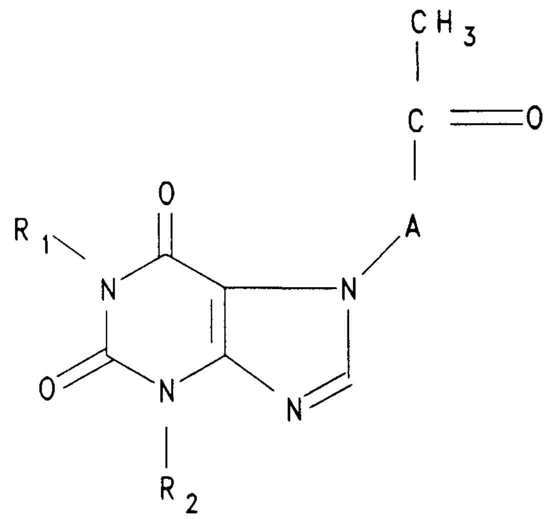

in which $R_1$ and $R_2$ are the same or different and are independently selected from the group consisting of straight-chain or branched-chain alkyl radicals with 2 to 6 carbon atoms, cyclohexyl, straight-chain or branched-chain alkoxyalkyl, and hydroxylalkyl radicals, and A is a hydrocarbon radical with up to 4 carbon atoms which can be substituted by a methyl group, and

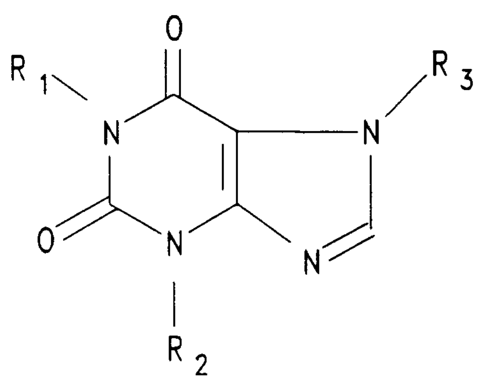

wherein at least one of $R_1$ and $R_3$ is either (a) a branched hydroxyalkyl group of the formula

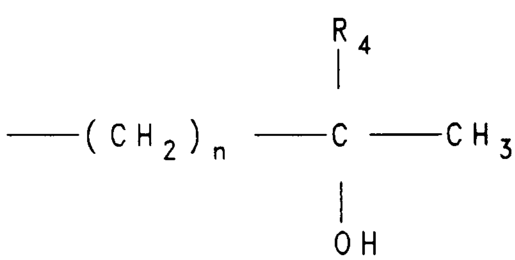

with a tertiary alcohol function, in which $R_4$ stands for an alkyl group with 1 to 3 carbon atoms and n stands for a whole number from 2 to 5,
the other $R_1$ or $R_3$ group that may optionally be present stands for a hydrogen atom or an aliphatic hydrocarbon group $R_5$ with up to 6 carbon atoms, whose carbon chain may be interrupted by up to 2 oxygen atoms or may be substituted with a hydroxy or oxo group, or

(b) at least one of $R_1$ or $R_3$ is an oxoalkyl group of the formula

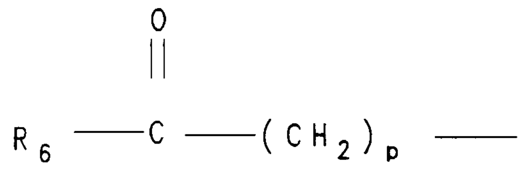

wherein $R_6$ is $C_1$-$C_6$ alkyl, and p is 2, 3 or 4;
the other $R_1$ or $R_3$ being defined as above and $R_2$ represents an alkyl group with 1 to 4 carbon atoms.

**4.** A process according to claim 3, wherein the compound is pentoxifylline.

**5.** A process according to one or more of claims 1 to 4, wherein the binder is a polymer selected from the group consisting of polyvinylpyrrolidone, a starch, a cellulose, and an oligosaccharide.

**6.** A process according to one or more of claims 1 to 5, wherein the number of discrete layers of a coating is from about 2 to about 25.

**7.** A process according to one or more of claims 1 to 6, having a pellet size of about 0.42 millimeters to about 3.36 millimeters.

**8.** A process according to one or more of claims 1 to 7, wherein the weight of medicament is from about 52 % to about 92 % of the weight of the micropellet.

European Patent Office

# EUROPEAN SEARCH REPORT

Application Number

EP 92 10 9699

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
| --- | --- | --- | --- |
| Y | US-A-3 400 185 (KENNETH H. KOHNLE) 3 September 1968<br>* column 1, line 31 - line 35 *<br>* column 4, line 3 - line 11 *<br>* column 4, line 22 - line 36 *<br>* column 4, line 71 - line 75 *<br>* column 5, line 9 - line 72 *<br>* example 1 *<br>--- | 1-8 | A61K 31/52<br>A61K9/50<br>A61K9/54<br>A61K9/52 |
| Y | WO-A-8 804 928 (HOECHST-ROUSSEL PHARMACEUTICALS INCORPORATED) 17 July 1988<br>* claims 1,3 *<br>* page 26, line 32 - page 27, line 12 *<br>----- | 1-8 | |
| | | | TECHNICAL FIELDS SEARCHED (Int. Cl.5 ) |
| | | | A61K |

The present search report has been drawn up for all claims

| Place of search | Date of completion of the search | Examiner |
| --- | --- | --- |
| THE HAGUE | 28 SEPTEMBER 1992 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document